# EUROPÄISCHE PATENTSCHRIFT

(11) **EP 3 262 129 B1**
(45) Veröffentlichungstag und Bekanntmachung des Hinweises auf die Patenterteilung: **12.06.2019**
(21) Anmeldenummer: 16705967.4
(22) Anmeldetag: 24.02.2016
(51) Int. Cl.: C09J 4/00, C09J 4/06, C07D 493/04, C08F 222/10

(54) **VERFAHREN ZUR HERSTELLUNG VON ISOSORBIDETHOXYLATDI(METH)ACRYLAT**
METHOD FOR PRODUCING ISOSORBIDE ETHOXYLATE DIMETHACRYLATE
PROCÉDÉ DE PRÉPARATION DE DI(MÉTH)ACRYLATE D'ÉTHOXYLATE D'ISOSORBIDE

(30) Priorität: 26.02.2015 US 201562120917 P
(43) Veröffentlichungstag der Anmeldung: 03.01.2018
(73) Patentinhaber: BASF SE, 67056 Ludwigshafen am Rhein (DE)
(72) Erfinder: MISSKE, Andrea, 67346 Speyer (DE); FLEISCHHAKER, Friederike, 67065 Ludwigshafen (DE); FLECKENSTEIN, Christoph, 63579 Freigericht (DE); KALLER, Martin, 68163 Mannheim (DE); STENGEL, Ulrik, 69488 Birkenau (DE); BLANCHOT, Mathieu, 67245 Lambsheim (DE); STOER, Claudia, 40597 Düsseldorf (DE); NAIR, Ritesh, 69115 Heidelberg (DE)
(86) Internationale Anmeldenummer: PCT/EP2016/053857
(87) Internationale Veröffentlichungsnummer: WO 2016/135190

(56) Entgegenhaltungen:
- DE-A1- 4 131 458
- ZHILA VAZIFEHASL ET AL: "New Series of Dimethacrylate-Based Monomers on Isosorbide as a Dental Material: Synthesis and Characterization", INTERNATIONAL JOURNAL OF COMPOSITE M ATERIALS, Bd. 3, Nr. 4, 1. Januar 2013 (2013-01-01), Seiten 100-107, XP55268012, US ISSN: 2166-479X, DOI: 10.5923/j.cmaterials.20130304.03

## Beschreibung

Die Erfindung betrifft ein Verfahren zur Herstellung von Isosorbidethoxylatdi(meth)acrylat durch Umesterung von Alkyl(meth)acrylat mit Isosorbidethoxylat sowie die Verwendung von Isosorbidethoxylatdi(meth)acrylat.

E2BADMA (Dimethacrylat von mit jeweils 2 Ethylenoxid-Einheiten alkoxyliertem Bisphenol-A) und E3BADMA (Dimethacrylat von mit jeweils 3 Ethylenoxid-Einheiten alkoxyliertem Bisphenol-A) sind Bisphenol A-basierte Dimethacrylate, die als Vernetzer zum Beispiel in Coatings und Mischungen für Bohrlöcher zum Einsatz kommen. Sie zeichnen sich durch eine schnelle Reaktionszeit aus, die dazu führt, dass 2-Komponenten-Mischungen schon nach kurzer Zeit teillastfest sind und die Endzugfestigkeit nach wenigen Stunden erreicht ist. Des Weiteren besitzen sie eine hohe Polymerisatdichte mit sehr gutem Kohäsions-/Adhäsions-Verhältnis, eine gute Kristallisationsfestigkeit bei gleichzeitig niedrigem Schrumpf und geringer Versprödungstendenz sowie eine hohe Benetzungskraft, um ein gutes Kriechvermögen in Kapillarrisse zu gewährleisten.

DE 41 31 458 A1 offenbart Zweikomponenten-Klebemassen für die chemische Befestigungstechnik umfassend ein Kunstharz enthaltend das Di(meth)acrylat eines alkoxylierten Bisphenols sowie ein Härtungsmittel für das Kunstharz. Das Dokument International Journal of Composite Materials, Bd. 3, Nr. 4, 2013, Seiten 100-107 offenbart Isosorbidethoxylatdimethacrylat für Dentalanwendungen. Für bestimmte Anwendungen wird nach Bisphenol A-freien Vernetzern mit ähnlichem Eigenschaftsprofil gesucht.

Isosorbid ist ein Diol auf Basis nachwachsender Rohstoffe und bietet sich strukturell als Alternative zu Bisphenol A an, da es ein ähnlich starres Grundgerüst besitzt.

Eine Möglichkeit, Di(meth)acrylate von Isosorbid herzustellen, ist die Veresterung von Isosorbid mit (Meth)acrylsäure oder die Umesterung von Methylacrylat, Ethylacrylat oder Methylmethacrylat mit Isosorbid in Gegenwart geeigneter Katalysatoren. Beiden OH-Gruppen des Isosorbids sind sekundäre OH-Gruppen mit vergleichsweise geringer Reaktivität, wobei die relativen Reaktivitäten der beiden OH-Gruppen zudem sehr unterschiedlich sind. Somit ist ein unvollständiger Umsatz zu erwarten.

DE 2 317 226 A1 offenbart ein Verfahren zur Herstellung von (Meth)acrylsäureestern aus einem Gemisch von C₁₀-C₁₈-Alkanolen durch Umesterung von Methyl(meth)acrylat in Gegenwart von Titanalkoholat als Katalysator und 2,6-Di-tert.-butylparakresol (TBK) als Stabilisator. Dabei wird in Gegenwart von Aktivkohle gearbeitet. Nach Beendigung der Reaktion wird Wasser zugegeben, wodurch das Titanalkoholat zu Titanhydroxid/-oxid hydrolysiert wird, welches an die Aktivkohle adsorbiert. Der Feststoff wird abfiltriert und das Reaktionsprodukt einer Wasserdampfdestillation unterzogen.

WO 2009/080380 offenbart ein Verfahren zur Herstellung von Methacrylaten von C₆-C₂₂-Alkoholen durch Umesterung von Methyl(meth)acrylat mit den entsprechenden Alkoholen in Gegenwart von Titanalkoholat als Katalysator. In Beispiel 1 wird Methylmethacrylat mit 2-Ethylhexanol in Gegenwart von Hydrochinonmonomethylether (MEHQ) als Stabilisator und Tetraisopropyltitanat als Katalysator umgesetzt. Dabei wird ein azeotropes Gemisch aus Methanol/Methylmethacrylat abdestilliert. Nach Abdestillieren von nicht umgesetztem Methylmethacrylat wird das Katalysator enthaltende 2-Ethylhexylmethacrylat einer Reindestillation im Vakuum (ca. 30 mbar) unterworfen. Dabei wird 2-Ethylhexylmethacrylat mit einer Reinheit von 99,4 % erhalten.

Aufgabe der Erfindung ist es, ein Verfahren zur Herstellung von (Meth)acrylaten von Isosorbid bereitzustellen, bei dem nur in geringem Umfang Nebenprodukte gebildet werden.

Gelöst wird die Aufgabe durch ein Verfahren zur Herstellung von Isosorbidethoxylatdi(meth)-acrylat durch Umesterung von Alkyl(meth)acrylat mit Isosorbidethoxylat, umfassend die Schritte:
(i) Ethoxylierung von Isosorbid zu Isosorbidethoxylat,
(ii) Reagieren lassen von Alkyl(meth)acrylat mit Isosorbidethoxylat in Gegenwart von Kaliumphosphat als Katalysator und eines Stabilisators in Gegenwart eines Schleppmittels, das mit dem in dem Alkyl(meth)acrylat gebundenen Alkohol ein Azeotrop bildet,
(iii) kontinuierliches Abdestillieren des Azeotrops aus Schleppmittel und Alkohol,
   wobei die Schritte (ii) und (iii) gleichzeitig durchgeführt werden, bis Isosorbidethoxylat im Wesentlichen vollständig umgesetzt ist,
(iv) Abtrennung des Katalysators von dem Isosorbidethoxylatdi(meth)acrylat enthaltenden Produktgemisch,
(v) Abdestillieren von nicht umgesetztem Alkyl(meth)acrylat und Schleppmittel aus dem Produktgemisch.

Überraschenderweise wurde gefunden, dass durch Umesterung von Alkyl(meth)acrylat mit Isosorbidethoxylat in Gegenwart von Kaliumphosphat als Katalysator Isosorbidethoxylatdi(meth)-acrylat in hohen Ausbeuten gebildet wird.

Durch Ethoxylierung von Isosorbid zu Isosorbidethoxylat werden die reaktionsträgen sekundären OH-Gruppen unterschiedlicher Reaktivität des Isosorbids teilweise in reaktive primäre OH-Gruppen von im Wesentlichen gleicher Reaktivität überführt. Diese reagieren in Gegenwart von Kaliumphosphat als Katalysator sehr gut mit Alkyl(meth)acrylat zu Isosorbidethoxylat-di(meth)acrylat.

Bei der Alkoxylierung mit nur wenigen Äquivalenten EO (zum Beispiel 3 Äquivalenten EO) wird ein Alkoholgemisch erhalten, das noch immer einen Anteil sekundärer OH-Gruppen besitzt. Wird EO in größerem molaren Verhältnis eingesetzt, steigt der Anteil an primärem Alkohol, es entstehen aber auch längere Polyalkylenoxidketten. Hierdurch werden die Eigenschaften des Vernetzers beeinflusst, der dann über eine flexiblere Struktur verfügt.

Der Umsetzungsgrad des durch die Alkoxylierung entstandenen Alkoholgemischs kann am einfachsten über die Bestimmung der OH-Zahl analysiert werden. Diese gibt den Gehalt an OH-Gruppen als Summenparameter in der Einheit mg KOH/g Substanz an und kann unter Annahme einer bestimmten molaren Masse des Alkohols in Gewichtsprozent umgerechnet werden.
Der Gehalt an Alkoholen, bestimmt über die OH-Zahl und beispielsweise berechnet als Isosorbid*3EO, in dem nach Schritt (v) erhaltenen Produkt beträgt vorzugsweise <2 Gew.-%, besonders bevorzugt <1 Gew.-%.

Daneben kann das nach Schritt (v) erhaltene Produkt noch Spuren von Schleppmittel und Alkyl(meth)acrylat enthalten. Diese können in Mengen von insgesamt bis zu 2 Gew.-%, bevorzugt bis zu 1 Gew.-% in dem nach Schritt (v) erhaltenen Produkt enthalten sein.

Die Menge aller Nebenkomponenten (Isosorbidethoxylat, Monoester des Isosorbidethoxylats, Schleppmittel und Alkyl(meth)acrylat, wobei Isosorbidethoxylat und Monoester des Isosorbidethoxylats über die OH-Zahl bestimmt werden und als Isosorbid*3EO berechnet werden) an dem nach Schritt (v) erhaltenen Produkt beträgt im Allgemeinen bis zu 4 Gew.-%, bevorzugt bis zu 2 Gew.-%.

In einem ersten Schritt (i) wird Isosorbid mit Ethylenoxid zu Isosorbidethoxylat ethoxyliert. Im Allgemeinen werden pro mol Isosorbidethoxylat 2 bis 4 mol Ethylenoxid, vorzugsweise 2 bis 3,5 mol Ethylenoxid umgesetzt.

Die Ethoxylierung kann mit gasförmigem Ethylenoxid in der Gegenwart von basischen oder sauren Katalysatoren bei einem Druck von 100 bis 500 kPa und vorzugsweise bei Temperaturen von 120 bis 220°C, wie beispielsweise in EP 2 174 941 beschrieben, durchgeführt werden. Geeignete basische Katalysatoren sind beispielsweise NaOH oder KOH oder Natrium- oder Kaliummethylat.

In Schritt (ii) wird Alkyl(meth)acrylat mit Isosorbidethoxylat in Gegenwart von Kaliumphosphat als Katalysator und eines Stabilisators in Gegenwart eines Schleppmittels, das mit dem in dem Alkyl(meth)acrylat gebundenen Alkohol ein Azeotrop bildet, reagieren gelassen, wobei gleichzeitig in Schritt (iii) das Azeotrop aus Schleppmittel und Alkohol abdestilliert wird, bis Isosorbidethoxylat im Wesentlichen vollständig umgesetzt ist. Die Umesterung besteht also aus den Schritten (ii) und (iii).

Geeignete Alkyl(meth)acrylate sind die C₁-C₄-Alkyl(meth)acrylate. Im Allgemeinen wird das Methyl(meth)acrylat oder Ethyl(meth)acrylat eingesetzt, wobei bei der Umesterungsreaktion Methanol oder Ethanol als Alkohole freigesetzt werden.

Die Reaktion von Alkyl(meth)acrylat mit Isosorbidethoxylat erfolgt in Gegenwart von festem, suspendiertem Kaliumphosphat als Katalysator.

Die Reaktion von Alkyl(meth)acrylat mit Isosorbidethoxylat erfolgt weiterhin in Gegenwart eines oder mehrerer Stabilisatoren (Polymerisationsinhibitoren). Geeignete Stabilisatoren können beispielsweise N-Oxide (Nitroxyl- oder N-Oxyl-Radikale, also Verbindungen, die wenigstens eine N-O-Gruppe aufweisen), wie z. B. 4-Hydroxy-2,2,6,6-tetramethylpiperidin-N-oxyl, 4-Oxo-2,2,6,6-tetramethylpiperidin-N-oxyl, 4-Acetoxy-2,2,6,6-tetramethyl-piperidin-N-oxyl, 2,2,6,6-Tetramethylpiperidin-N-oxyl, Bis(1-oxyl-2,2,6,6-tetramethylpiperidine-4-yl)sebacat, 4,4',4"-Tris(2,2,6,6-tetramethyl-piperidin-N-oxyl)-phosphit oder 3-Oxo-2,2,5,5-tetramethylpyrrolidin-N-oxyl; ein- oder mehrwertige Phenole, die ggf. eine oder mehrere Alkylgruppen aufweisen, wie zum Beispiel Alkylphenole, beispielsweise o-, m- oder p-Kresol (Methylphenol), 2-tert.-Butylphenol, 4-tert.-Butylphenol, 2,4-Di-tert.-butylphenol, 2-Methyl-4-tert.-butylphenol, 2-tert.-Butyl-4-methylphenol, 2,6-tert.-Butyl-4-methylphenol, 4-tert.-Butyl-2,6-dimethylphenol oder 6-tert.-Butyl-2,4-dimethylphenol; Chinone, wie zum Beispiel Hydrochinon, Hydrochinonmonomethylether, 2-Methylhydrochinon oder 2,5-Di-tert.-Butylhydrochinon; Hydroxyphenole, wie beispielsweise Brenzcatechin (1,2-Dihydroxybenzol) oder Benzochinon; Aminophenole, wie zum Beispiel p-Aminophenol; Nitrosophenole, wie zum Beispiel p-Nitrosophenol; Alkoxyphenole, wie beispielsweise 2-Methoxyphenol (Guajacol, Brenzcatechinmonomethylether), 2-Ethoxyphenol, 2-Isopropoxyphenol, 4-Methoxyphenol (Hydrochinonmonomethylether), Mono- oder Di-tert.-Butyl-4-methoxyphenol; Tocipherole, wie zum Beispiel α-Tocopherol sowie 2,3-Dihydro-2,2-dimethyl-7-hydroxybenzofuran (2,2-Dimethyl-7-hydroxycumaran), aromatische Amine, wie zum Beispiel N,N-Diphenylamin oder N-Nitrosodiphenylamin; Phenylendiamine, wie zum Beispiel N,N'-Dialkyl-p-phenylendiamin, wobei die Alkylreste gleich oder verschieden sein können und jeweils unabhängig voneinander aus 1 bis 4 Kohlenstoffatomen bestehen und geradkettig oder verzweigt sein können, wie zum Beispiel N,N'-Dimethyl-p-phenylendiamin oder N,N'-Diethyl-p-phenylendiamin, Hydroxylamine, wie zum Beispiel N,N-Diethylhydroxylamin, Imine, wie zum Beispiel Methylethylimin oder Methylen violett, Sulfonamide, wie zum Beispiel N-Methyl-4-toluolsulfonamid oder N-tert.-Butyl-4-toluolsulfonamid, Oxime, wie Aldoxime, Ketoxime oder Amidoxime, wie zum Beispiel Diethylketoxim, Methylethylketoxim oder Salicyladoxim, phosphorhaltige Verbindungen, wie zum Beispiel Triphenylphosphin, Triphenylphosphit, Triethylphosphit, hypophsophorige Säure oder Alkylester der Phosphorigen Säuren; schwefelhaltige Verbindungen wie zum Beispiel Diphenylsulfid oder Phenothiazin oder Gemische davon sein.

Bevorzugt sind Hydrochinon, Hydrochinonmonomethylether, Phenothiazin, 4-Hydroxy-2,2,6,6-tetramethylpiperidin-N-oxyl, 4-Oxo-2,2,6,6-tetramethylpiperidin-N-oxyl, 2-tert.-Butylphenol, 4-tert.-Butylphenol, 2,4-Di-tert.-butylphenol, 2-tert.-Butyl-4-methylphenol, 6-tert.-Butyl-2,4-dimethylphenol, 2,6-Di-tert.-Butyl-4-methylphenol und 2-Methyl-4-tert.-butylphenol.

Besonders bevorzugt ist Hydrochinonmonomethylether (MeHQ).

Vorteilhaft kann zusätzlich als Polymerisationsinhibitor Sauerstoff eingesetzt werden.

Zur weiteren Stabilisierung kann ein sauerstoffhaltiges Gas, bevorzugt Luft oder ein Gemisch aus Luft und Stickstoff (Magerluft) anwesend sein.

Die Umesterungsreaktion (Schritte (ii) und (iii)) wird im Allgemeinen bei einer Temperatur von 60 bis 140°C, bevorzugt 70 bis 110°C durchgeführt. Dabei wird ein Azeotrop aus Schleppmittel und Alkohol kontinuierlich abdestilliert.

Geeignete, mit Methanol oder Ethanol ein azeotrop siedendes Gemisch bildende Schleppmittel sind zunächst Methylacrylat und Methylmethacrylat sowie Ethylacrylat und Ethylmethacrylat selbst. Als separate Schleppmittel geeignet sind unter anderem Cyclohexan, Methylcyclohexan, Benzol, Toluol, Hexane und Heptane und Mischungen hieraus. Bevorzugt sind Methylacrylat, Methylmethacrylat, Ethylacrylat und Ethylmethacrylat sowie Mischungen von diesen mit n-Heptan und Cyclohexan. Der Begriff Schleppmittel umfasst in diesem Sinne das Edukt selbst sowie gegebenenfalls ein zusätzlich eingesetztes separates Lösungsmittel.

In einer bevorzugten Ausführungsform wird kein separates Lösungsmittel als Schleppmittel eingesetzt. In diesem Fall fungiert das Edukt Alkyl(meth)acrylat selbst als Schleppmittel.

Das Schleppmittel kann anschließend wieder im Reaktor ergänzt werden. Hierzu wird das azeotrope Gemisch aus Alkohol und Schleppmittel in einer bevorzugten Ausführungsform über eine geeignete Kolonne abdestilliert, in einem Mischgefäß mit Wasser gerührt und dann in einen Phasenseparator überführt, wobei sich der Alkohol, im Allgemeinen Methanol oder Ethanol, in Wasser löst und sich die organische Phase als obere Schicht abscheidet. Die organische Phase wird vorzugsweise über den Kopf der Kolonne dem Reaktionsgemisch wieder zugeführt und damit bis auf geringe Verluste im Kreislauf geführt. Es kann aber auch alternativ frisches Schleppmittel hinzugeführt werden und eine Aufarbeitung des Schleppmittel-Alkohol-Gemisches in einem separaten Schritt erfolgen oder auf die Ergänzung des Schleppmittels ganz oder teilweise verzichtet werden.

Im Allgemeinen wird Alkyl(meth)acrylat in stöchiometrischem Überschuss eingesetzt. Vorzugsweise beträgt der Überschuss an Methyl(meth)acrylat pro zu veresternder Hydroxylgruppe 5 bis 1000 mol-%, besonders bevorzugt 50 bis 500 mol-%, insbesondere 100 bis 400 mol-%.

Der Katalysator wird in einer Konzentration von 0,1-10 mol-%, bezogen auf die Menge an Isosorbidethoxylat, eingesetzt, bevorzugt in einer Konzentration von 0,1 bis 5 mol-%.

Die Umesterung kann bei Atmosphärendruck, aber auch bei Überdruck oder Unterdruck durchgeführt werden. Im Allgemeinen wird sie bei 300 bis 1000 mbar, bevorzugt bei 300 bis 700 mbar (Atmosphärendruck = 1000 mbar) durchgeführt. Die Reaktionszeit beträgt im Allgemeinen 1 bis 24 Stunden, vorzugsweise 3 bis 18 Stunden, besonders bevorzugt 3 bis 10 Stunden. Die Umesterung (Schritte (ii) und (iii)) kann kontinuierlich, beispielsweise in einer Rührkesselkaskade, oder diskontinuierlich erfolgen.

Die Reaktion kann in allen für eine solche Umsetzung geeigneten Reaktoren durchgeführt werden. Solche Reaktoren sind dem Fachmann bekannt. Bevorzugt erfolgt die Umsetzung in einem Rührkesselreaktor.

Zur Durchmischung des Ansatzes können beliebige Vorrichtungen eingesetzt werden wie zum Beispiel Rührvorrichtungen. Die Durchmischung kann auch durch Einspeisen eines Gases, vorzugsweise eines Sauerstoff-haltigen Gases erfolgen.

Das Entfernen des gebildeten Alkohols, im Allgemeinen Methanol oder Ethanol, erfolgt kontinuierlich oder schrittweise in an sich bekannter Weise durch azeotrope Destillation in Gegenwart eines Schleppmittels. Zusätzlich kann Methanol auch durch Strippen mit einem Gas entfernt werden.

In einer bevorzugten Ausführungsform wird aus dem in Schritt (iii) abdestillierten Azeotrop aus Schleppmittel und Alkohol der Alkohol durch Waschen mit Wasser abgetrennt und das Schleppmittel in den Reaktionsbehälter zurückgeführt.

Die Schritte (ii) und (iii) werden durchgeführt, bis das eingesetzte Isosorbidethoxylat im Wesentlichen vollständig umgesetzt ist. Dies ist der Fall, wenn Isosorbidethoxylat zu 95 %, bevorzugt zu 97 %, besonders bevorzugt zu 98 % zum Diester umgesetzt ist. Der Umsetzungsgrad kann am einfachsten über die Bestimmung der OH-Zahl analysiert werden. Diese gibt den Gehalt an OH-Gruppen als Summenparameter in der Einheit mg KOH/g Substanz an und kann unter Annahme einer bestimmten molaren Masse des Alkohols in Gewichtsprozent umgerechnet werden.

In Schritt (iv) wird der feste Katalysator von dem Isosorbidethoxylatdi(meth)acrylat enthaltenden Produktgemisch abgetrennt, zum Beispiel durch Filtration oder Zentrifugation.

Die Filtration kann beispielsweise mit einer Druckfilternutsche durchgeführt werden. Verfahrenstechnisch können für eine Filtration im erfindungsgemäßen Verfahren alle an sich bekannten Filtrationsverfahren und -apparate eingesetzt werden, zum Beispiel solche, die in Ullmann's Encyclopedia of Industrial Chemistry, 7th Ed. 2013 Electronic Release, Kapitel: Filtration, 1. Fundamentals und Filtration 2. Equipment, beschrieben sind. Beispielsweise können dies Kerzenfilter, Filterpressen, Tellerdruckfilter, Beutelfilter oder Trommelfilter sein. Vorzugsweise werden Kerzenfilter oder Tellerdruckfilter eingesetzt. Die Filtration kann mit oder ohne Filterhilfsmittel durchgeführt werden. Geeignete Filterhilfsmittel sind Filterhilfsmittel basierend auf Kieselgur, Perlit und Cellulose.

Geeignete Zentrifugen und auch Separatoren sind dem Experten bekannt. Verfahrenstechnisch können für eine Zentrifugation im erfindungsgemäßen Verfahren alle an sich bekannten Zentrifugationsverfahren und -apparate eingesetzt werden, zum Beispiel solche, die in Ullmann's Encyclopedia of Industrial Chemistry, 7th Ed. 2013 Electronic Release, Kapitel: Centrifuges, Filtering und Centrifuges, Sedimenting, beschrieben sind.

Die Abtrennung des Katalysators kann auch als wässrige Extraktion durch Zugabe von Wasser erfolgen.
Hierzu wird das Produktgemisch, das noch nicht umgesetztes Alkyl(meth)acrylat und gegebenenfalls separates Schleppmittel sowie den Stabilisator und den Katalysator enthält, mit Wasser in Kontakt gebracht. Es können auch mehrere Waschschritte, beispielsweise 3 Waschschritte, durchgeführt werden. Die Waschwassermenge beträgt pro Waschschritt im Allgemeinen das 0,1 bis 2-fache, bevorzugt das 0,2 bis 0,5-fache des Produktgemischs.

Die Wäsche kann beispielsweise in einem Rührbehälter oder in einer anderen herkömmlichen Apparatur, zum Beispiel in einer Kolonne oder Mixer-Settler-Apparatur durchgeführt werden.

Verfahrenstechnisch können für eine Wäsche im erfindungsgemäßen Verfahren alle an sich bekannten Extraktions- und Waschverfahren und -apparate eingesetzt werden, zum Beispiel solche, die in Ullmann's Encyclopedia of Industrial Chemistry, 6th Ed. 1999 Electronic Release, Kapitel "Liquid - Liquid Extraction - Apparatus" beschrieben sind. Beispielsweise können dies ein- oder mehrstufige, bevorzugt einstufige Extraktionen sowie Extraktionen in Gleich- oder Gegenstromfahrweise sein.

Das gewaschene Reaktionsgemisch wird gegebenenfalls mit einem Lagerstabilisator versetzt, so dass im Zielprodukt die gewünschte Konzentration an Stabilisator erreicht wird, beispielsweise 100 ppm. Diese mit diesem Verfahren beliebig einstellbare Konzentration ist von der jeweiligen Spezifikation des Endproduktes abhängig und liegt zum Beispiel für kommerzielle Alkyl(meth)acrylate im Bereich von 15 bis 200 ppm. Als Lagerstabilisator dienen in der Regel Stabilisatoren, ausgewählt aus der Gruppe der Phenole wie zum Beispiel 2,6-Di-tert.-butyl-4-methylphenol, 6-tert.-Butyl-2,4-dimethylphenol, Hydrochinon und Hydrochinonmonomethylether, bevorzugt Hydrochinonmonomethylether.

Anschließend werden in einem Destillationsschritt (v) nicht umgesetztes Alkyl(meth)acrylat und gegebenenfalls separates Schleppmittel sowie gegebenenfalls Wasser aus dem Produktgemisch abdestilliert. Diese Destillation erfolgt im Allgemeinen bei einer Temperatur von 40 bis 100°C, bevorzugt 60 bis 80°C und einem variablen Druck von 2 bis 700 mbar. Zusätzlich können diese Komponenten auch durch Strippen mit einem Gas, vorzugsweise einem sauerstoff-haltigen Gas, entfernt werden.

Die destillative Abtrennung erfolgt beispielsweise in einem Rührkessel mit Doppelwandheizung und/oder innenliegenden Heizschlangen unter vermindertem Druck.

Selbstverständlich kann die Destillation auch in einem Fallfilm- oder Dünnschichtverdampfer erfolgen. Dazu wird das Reaktionsgemisch, bevorzugt mehrmals im Kreislauf, unter vermindertem Druck, beispielsweise bei 20 bis 700 mbar, bevorzugt 30 bis 500, besonders bevorzugt 50 bis 150 mbar und einer Temperatur von 40 bis 80°C durch den Apparat geführt.

Vorteilhaft kann ein inertes Gas, bevorzugt ein sauerstoffhaltiges Gas, besonders bevorzugt Luft oder ein Gemisch aus Luft und Stickstoff (Magerluft) in den Destillationsapparat eingeleitet werden, beispielsweise 0,1 bis 1, bevorzugt 0,2 bis 0,8 und besonders bevorzugt 0,3 bis 0,7 m³/m³h, bezogen auf das Volumen des Reaktionsgemisches.

Nach Durchführung der Schritte (iv) und (v) verbleibt ein Produkt als Sumpfprodukt, welches die oben beschriebene Reinheit aufweist.

Gegenstand der Erfindung ist auch die Verwendung von Isosorbiddi(meth)acrylat als Harzkomponente für Zweikomponenten-Klebemassen.

Erfindungsgemäße Zweikomponentenklebmassen für die chemische Befestigungstechnik enthalten
I. ein Kunstharz mit einer Viskosität bei 23°C zwischen 100 und 10 000 (mPa•s), welches das Isosorbiddi(meth)acrylat enthält, und
II. ein Härtungsmittel für das Kunstharz.

Die eine Komponente der erfindungsgemäßen Klebmasse ist ein Kunstharz mit einer Viskosität (bei 23°C) zwischen 100 und 10 000, vorzugsweise 200 bis 2000 und insbesondere 500 bis 1500 mPa•s, gemessen in Abwesenheit von Füllstoffen. Es enthält das Isosorbiddi(meth)arylat.

Das Kunstharz kann 2 bis 20 Gew.-% anderer härtbarer Harze, wie Polyester-, Vinylester-, Bismaleinimid- oder Epoxid-Harze, sowie zum Zweck der Zähmodifizierung 2 bis 20 Gew.-% eines Thermoplasten, wie Polyamid, Polyester, oder eines Kautschuks enthalten.

Falls für die Peroxidhärtung Beschleuniger erforderlich sind, werden diese zweckmäßigerweise räumlich zusammen mit dem Harz, d.h. getrennt vom Härter, angeordnet. Geeignete Beschleuniger sind: Aromatische Amine wie N,N-Dimethylanilin, N,N-Diethylanilin; Toluidine und Xylidine wie N,N- Diisopropyliden-para-toluidin, N,N-Dimethyl-p-toluidin, N,N- Bis-(2-hydroxyethyl)-xylidin; ferner Co-, Mn-, Sn- oder Ce-Salze, wie zum Beispiel Cobaltnaphthenat, sowie Mischungen von Amin- und Cobaltbeschleunigern. Die Beschleuniger sind im Kunstharz im Allgemeinen in Mengen von vorzugsweise 0,5 bis 5 Gew.-% enthalten.

In der Regel wird die Konfektionsform einer 2-Kammerpatrone gewählt. Als Kartuschen werden vorzugsweise Zweikammerkartuschen verwendet, deren größere Kammer das Harz und die kleinere Kammer das Härtungsmittel enthält. Die größere Kammer hat ein etwa 5 bis 10 mal größeres Volumen als die kleinere Kammer.

In der Kammer, welche die Kunstharzkomponente enthält, können daneben auch noch Füllstoffe vorhanden sein. Als verstärkende Füllstoffe für die Klebmasse dienen zum Beispiel Quarz, Glas, Korund, Porzellan, Steingut, Schwerspat, Leichtspat, Talkum und Kreide. Die Füllstoffe werden in Form von Sanden, Mehlen oder speziellen Formkörpern (Zylinder, Kugeln usw.) entweder der Harzlösung und/oder dem Härter (Initiator) zugemischt. Die Füllstoffe können als Fasern (fibrilläre Füllstoffe) eingesetzt werden. Bevorzugt und deutlicher verstärkend wirken sich die globulären, inerten Stoffe (Kugelform) aus.

Räumlich getrennt vom Harz ist das Härtungsmittel vorgesehen. Bevorzugte Härtungsmittel sind bei niedrigen Temperaturen zerfallende organische Peroxide. Besonders gut geeignet sind Benzoylperoxid und Methylethylketonperoxid, ferner tert.-Butylperbenzoat, Cyclohexanonperoxid, Laurylperoxid und Cumolhydroperoxid, sowie Mischungen verschiedener Peroxide. Die Peroxide werden vorzugsweise in Mengen von 0,5 bis 10 Gew.-%, vorzugsweise von 1 bis 5 Gew.-% eingesetzt. Die Härtungsmittel werden zweckmäßigerweise auf inerte Füllstoffe aufgebracht, wobei Quarzsande mit Korngrößen von 0,5 bis 3 mm oder von 3 bis 6 mm bevorzugt sind.

Im Fall schaumfähiger Klebemassen wird zweckmäßigerweise Carbonat zum Harz gegeben, die Säure-Komponente kann entweder mit dem Härter zusammen in eine Kammer oder aber in eine separate dritte Kammer gefüllt werden.

Die erfindungsgemäße Zweikomponenten-Klebmasse kann als Dübelmasse zur Befestigung von Verankerungen in Bohrlöchern eingesetzt werden. Derartige Verankerungen weisen ein gutes Rissdehnungsverhalten, geringe Schrumpfspannung und ausgezeichnete Adhäsion an mineralischen Aufnahmewerkstoffen, wie Beton und Naturstein, sowie an Schaum- und Hohlblocksteinen auf.

Die Erfindung wird durch die nachstehenden Beispiele näher ausgeführt.

### Beispiele

### Beispiel 1

Isosorbid (5110 g, 35.0 mol) wurde mit 3 Äquivalenten Ethylenoxid (4620 g, 105 mol) und katalytischen Mengen KOH (48,3 g, 0,863 mol) bei 160 bis 180°C in einem Druckreaktor bei maximal 5 bar umgesetzt. Nach beendeter Reaktion wurde das Produkt abgekühlt und mit Essigsäure neutralisiert.

### Beispiel 2

### Umesterung von Isosorbid*3EO in Gegenwart von Kaliumphosphat:

Die Umesterung erfolgte unter Lufteinleitung in einem 750 mL-Doppelmantelreaktor, ausgestattet mit einem Anker-Rührer, einer Lufteinleitung, einer Trennkolonne und einem Flüssigkeitsteiler. In dieser Apparatur wurden 200 g Isosorbid*3EO (OH-Zahl 415 mg KOH/g), 0,06 g Methylhydrochinon (MEHQ) und 600 g Methylmethacrylat (MMA, stabilisiert mit 15 ppm MEHQ) bei Raumtemperatur vorgelegt. 9,4 g Kaliumphosphat wurden zugegeben und das Reaktionsgemisch bei einer Badtemperatur von 80°C aufgeheizt, welche im Verlauf der Reaktion auf 100°C angepasst wurde. Es wurde ein Druck von 300 mbar, später 400 mbar (abs.) eingestellt und kontinuierlich ein Azeotrop aus Methanol und MMA abdestilliert, wobei die Sumpftemperatur von 60 auf 80°C anstieg. Das Rücklaufverhältnis betrug 2:1, später 10:1 (Rücklauf: Ablauf). Nach Beenden der Reaktion wurde das Produkt über einen Papierfilter filtriert und das Reaktionsgemisch im Vakuum konzentriert. Das Reaktionsprodukt weist eine OH-Zahl von 2 mg KOH/g auf, entsprechend < 0,5 % Alkohol, berechnet als Restalkohol Isosorbid*3EO, oder 1,2 %, berechnet als Monomethacrylsäureester von Isosorbid*3EO (alle Angaben sind Gew.-%, sofern nicht anders angegeben).

### Vergleichsbeispiel 1

### Umesterung von Isosorbid in Gegenwart von Kaliumphosphat:

Die Umesterung erfolgte in einem 1,6 L-Doppelmantelreaktor, ausgestattet mit einem Anker-Rührer, einer Lufteinleitung, einer Trennkolonne und einem Flüssigkeitsteiler. Das Rücklaufverhältnis betrug 10:1, später 10:3 (Rücklauf: Ablauf), die Rührgeschwindigkeit 180 U/min und die Lufteinleitung 1,5 L/h. In dieser Apparatur wurden 175 g Isosorbid, 0,48 g Methylhydrochinon (MEHQ) und 1200 g Methylmethacrylat (MMA, stabilisiert mit 15 ppm MEHQ) bei Raumtemperatur vorgelegt. 19,1 g Kaliumphosphat wurden zugegeben und das Reaktionsgemisch bei einer Badtemperatur von 80°C aufgeheizt, welche im Verlauf der Reaktion auf 100°C angepasst wurde. Es wurde ein Druck von 400 mbar (abs.) eingestellt und kontinuierlich ein Azeotrop aus Methanol und MMA abdestilliert, wobei die Sumpftemperatur von 75 auf 82°C anstieg. Nach Beenden der Reaktion wurde das Produkt über einen Papierfilter filtriert und das Reaktionsgemisch im Vakuum konzentriert. Das Reaktionsgemisch hatte folgende Zusammensetzung (in GC FI%): Isosorbid (0 %), Summe Monomethacrylate (4,3 %), Summe Nebenprodukte 10 % (nicht weiter analysiert), Zielprodukt Dimethacrylat (> 85,7 %).

### Vergleichsbeispiel 2

### Veresterung von Isosorbid*3EO:

In einem 1L-Vierhalskolben, ausgestattet mit Thermometer, Rührer, Wasserauskreiser und Lufteinleitung wurden Cyclohexan (96 g), Isosorbid*3EO (271 g; OH-Zahl 415 mg KOH/g), MeHQ (0,34 g), hypophosphorige Säure 50 %ig (0,84 g) und Cu(II)Acetat-Lösung (5 %ig, 3 g) vorgelegt. Anschließend wurde Methacrylsäure zudosiert (120 g, stabilisiert mit 200 ppm MeHQ). Methansulfonsäure (9,6 g) wurde hinzugefügt. Es wurde aufgeheizt. Nach 3 h 40 min wurden weitere 69 g Methacrylsäure zudosiert. Im Verlauf der Reaktion wurde ein Teil des Cyclohexans destillativ entfernt. Bei einer Innentemperatur von 83 bis 108°C ging Wasser über. Nach 12 h wurde die Reaktion abgebrochen. Die Reaktionsmischung wurde nach dem Abkühlen mit 200 mL Cyclohexan versetzt und mit 15 %iger Kochsalzlösung, mit NaOH-Lösung und noch einmal mit 15 %iger Kochsalzlösung extrahiert. Nach Phasentrennung wurde die organische Phase im Vakuum konzentriert. Das Reaktionsprodukt wies eine OH-Zahl von 16 mg KOH/g auf, entsprechend 3,9% Alkohol berechnet als Restalkohol Isosorbid*3EO, oder 9,7 %, berechnet als Monomethacrylsäureester von Isosorbid*3EO.

### Vergleichsbeispiel 3

### Umesterung von Isosorbid*3EO in Gegenwart eines Ti-Katalysators:

In einem 0,75L-Planschliffreaktor mit Kolonne, Kühler, Flüssigkeitsteiler, Ankerrührer sowie Lufteinleitung wurden Ethylacrylat (750 g), MeHQ (0,4 g), PTZ (0,04 g) sowie Isosobid*3EO (340 g; OH-Zahl 415,5 mg KOH/g) vorgelegt und unter Lufteinleitung und Rühren bei einer Badtemperatur von 95°C hochgeheizt. Bei einem Druck von 300 mbar wurden 105 g Ethylacrylat (EA) abdestilliert. 105 g Ethylacrylat sowie Titantetraisopropoxylat (14,3 g) wurden zudosiert und weiter auf 92°C Sumpftemperatur aufgeheizt. Die Reaktionsmischung war zunächst trüb, später klar. Ethylacrylat wurde mit einem Rücklaufverhältnis von 3:1 abdestilliert. EA wurde portionsweise zudosiert in den Mengen, die dem EA im Destillat entsprachen. Die Sumpftemperatur stieg im Verlauf der Reaktion auf 102°C an. In regelmäßigen Abständen wurden Sumpf- und Destillatproben gezogen, um den Verlauf der Reaktion zu beobachten. Es wurde ein Vakuum von maximal 925 mbar angelegt. Nach 5 h wurde die Reaktion abgebrochen und der Ansatz abgekühlt.

Das Reaktionsgemisch wurde mit 25 mL Wasser versetzt, über einen Papierfilter abfiltriert und im Vakuum konzentriert.

Das Reaktionsprodukt wies eine OH-Zahl von 27 mg KOH/g auf, entsprechend 6,5 % Alkohol, berechnet als Restalkohol Isosorbid*3EO, oder 15,6 %, berechnet als Monoacrylsäureester von Isosorbid*3EO.

### Vergleichsbeispiel 4

### Umesterung von Isosorbid*3EO in Gegenwart eines Ti-Katalysators:

In einem 0,75L-Planschliffreaktor mit Kolonne, Kühler, Flüssigkeitsteiler, Ankerrührer sowie Lufteinleitung wurden Methylmethacrylat (750 g), MeHQ (0,26 g), PTZ (0,03 g) sowie Isosorbid*3EO (338 g, OH-Zahl 415,5 mg KOH/g) vorgelegt und unter Lufteinleitung und Rühren bei einer Badtemperatur von 95°C hochgeheizt. Bei einem Druck von 300 mbar wurden 105 g Methylmethacrylat abdestilliert. 105 g Methylmethacrylat sowie Titantetraisopropoxylat (14,2 g) wurden zudosiert und weiter auf 98°C Sumpftemperatur aufgeheizt. Die Reaktionsmischung war zunächst trüb, später klar. Methylmethacrylat wurde mit einem Rücklaufverhältnis von 10:3 später 5:3 abdestilliert. Methylmethacrylat wurde portionsweise zudosiert in den Mengen, die dem Methylmethacrylat im Destillat entsprachen. Die Sumpftemperatur stieg im Verlauf der Reaktion auf 105°C an. In regelmäßigen Abständen wurden Sumpf- und Destillatproben gezogen, um den Verlauf der Reaktion zu beobachten. Es wurde ein Vakuum von maximal 900 mbar angelegt. Nach 12 h wurde die Reaktion abgebrochen und der Ansatz abgekühlt.

Das Reaktionsgemisch wurde mit 30 ml Wasser versetzt, über einen Papierfilter abfiltriert und im Vakuum konzentriert.

Das Reaktionsprodukt weist eine OH-Zahl von 21 mg KOH/g auf, entsprechend 5,1 % Alkohol, berechnet als Restalkohol Isosorbid*3EO, oder 12,7 %, berechnet als Monomethacrylsäureester von Isosorbid*3EO.

### Vergleichsbeispiel 5

### Umesterung von Isosorbid*3EO in Gegenwart eines Sn-Katalysators:

In einem 0,75L-Planschliffreaktor mit Kolonne, Kühler, Flüssigkeitsteiler, Ankerrührer sowie Lufteinleitung wurden Ethylacrylat (481 g), MeHQ (0,61 g), PTZ (0,61 g), Cyclohexan (89 g), Dimethylzinndichlorid (0,46 g), 30 %ige Natriummethylatlösung in Methanol (0,32 g) sowie Isosorbid*3EO (406 g, OH-Zahl 415,5 mg KOH/g) vorgelegt und unter Lufteinleitung und Rühren auf eine Sumpftemperatur von 91°C hochgeheizt. Nach Siedebeginn wurde ein Rücklaufverhältnis von 20:1 eingestellt, das im Verlauf der Reaktion variiert wurde auf bis zu 2:1. Ethylacrylat und Cyclohexan werden portionsweise zudosiert, entsprechend der abdestillierten Mengen.

Die Sumpftemperatur stieg im Verlauf der Reaktion auf 106°C. In regelmäßigen Abständen wurden Sumpfproben gezogen, um den Verlauf der Reaktion zu beobachten.

Nach 21h wurde der Versuch abgebrochen. Das Reaktionsprodukt weist eine OH-Zahl von 44 mg KOH/g auf, entsprechend 10,6 % Alkohol, berechnet als Restalkohol Isosorbid*3EO, oder 25,5 %, berechnet als Monoacrylsäureester von Isosorbid*3EO.

## Patentansprüche

1. Verfahren zur Herstellung von Isosorbidethoxylatdi(meth)acrylat durch Umesterung von Alkyl(meth)acrylat mit Isosorbidethoxylat, umfassend die Schritte:
(i) Ethoxylierung von Isosorbid zu Isosorbidethoxylat,
(ii) Reagieren lassen von Alkyl(meth)acrylat mit Isosorbidethoxylat in Gegenwart von Kaliumphosphat als Katalysator und eines Stabilisators und in Gegenwart eines Schleppmittels, das mit dem in dem Alkyl(meth)acrylat gebundenen Alkohol ein Azeotrop bildet,
(iii) kontinuierliches Abdestillieren des Azeotrops aus Schleppmittel und Alkohol,
wobei die Schritte (ii) und (iii) gleichzeitig durchgeführt werden, bis Isosorbidethoxylat im Wesentlichen vollständig umgesetzt ist,
(iv) Abtrennung des Katalysators von dem Isosorbidethoxylatdi(meth)acrylat enthaltenden Produktgemisch,
(v) Abdestillieren von nicht umgesetztem Alkyl(meth)acrylat und Schleppmittel aus dem Produktgemisch.

2. Verfahren nach Anspruch 1, **dadurch gekennzeichnet, dass** das Schleppmittel das Alkyl(meth)acrylat ist.

3. Verfahren nach Anspruch 1, **dadurch gekennzeichnet, dass** das Schleppmittel ein separates, von Alkyl(meth)acrylat verschiedenes Lösungsmittel ist.

4. Verfahren nach Anspruch 3, **dadurch gekennzeichnet, dass** das Schleppmittel ausgewählt ist aus der Gruppe bestehend aus n-Heptan und Cyclohexan.

5. Verfahren nach einem der Ansprüche 1 bis 4, **dadurch gekennzeichnet, dass** das Alkyl(meth)acrylat das Methyl- oder Ethyl(meth)acrylat ist.

6. Verfahren nach einem der Ansprüche 1 bis 5, **dadurch gekennzeichnet, dass** der Stabilisator Hydrochinonmonomethylether ist.

7. Verfahren nach einem der Ansprüche 1 bis 6, **dadurch gekennzeichnet, dass** aus dem in Schritt (iii) abdestillierten Azeotrop aus Schleppmittel und Alkohol durch Waschen mit Wasser der Alkohol abgetrennt und das Schleppmittel in den Reaktionsbehälter zurückgeführt wird.

8. Verfahren nach einem der Ansprüche 1 bis 7, **dadurch gekennzeichnet, dass** nach Schritt (v) ein Isosorbidethoxylatdi(meth)acrylat mit einem Gehalt an Nebenkomponenten von < 4 Gew.-% erhalten wird.

9. Verfahren nach Anspruch 8, **dadurch gekennzeichnet, dass** nach Schritt (v) ein Isosorbidethoxylatdi(meth)acrylat mit einem Gehalt an Nebenkomponenten von < 2 Gew.-% erhalten wird.

10. Verwendung von Isosorbidethoxylatdi(meth)acrylat als Harzkomponente für Zweikomponenten-Klebemassen.

11. Verwendung nach Anspruch 10, **dadurch gekennzeichnet, dass** die Zweikomponenten-Klebemassen Dübelmassen zur Befestigung von Verankerungsmitteln in Bohrlöchern sind.

12. Verwendung nach Anspruch 10 oder 11, **dadurch gekennzeichnet, dass** das Isosorbidethoxylatdi(meth)acrylat nach dem Verfahren gemäß einem der Ansprüche 1 bis 9 hergestellt ist.

## Claims

1. A process for preparing isosorbide ethoxylate di(meth)acrylate by transesterifying alkyl (meth)acrylate with isosorbide ethoxylate, comprising the steps of:
(i) ethoxylating isosorbide to give isosorbide ethoxylate,
(ii) reacting alkyl (meth)acrylate with isosorbide ethoxylate in the presence of potassium phosphate as catalyst and a stabilizer and in the presence of an azeotroping agent which forms an azeotrope with the alcohol bound in the alkyl (meth)acrylate,
(iii) continuously distilling off the azeotrope of azeotroping agent and alcohol,
wherein steps (ii) and (iii) are conducted simultaneously until the isosorbide ethoxylate has been essentially fully converted,
(iv) removing the catalyst from the product mixture comprising isosorbide ethoxylate di(meth)acrylate,
(v) distilling unconverted alkyl (meth)acrylate and azeotroping agent out of the product mixture.

2. The process according to claim 1, wherein the azeotroping agent is the alkyl (meth)acrylate.

3. The process according to claim 1, wherein the azeotroping agent is a separate solvent other than alkyl (meth)acrylate.

4. The process according to claim 3, wherein the azeotroping agent is selected from the group consisting of n-heptane and cyclohexane.

5. The process according to any of claims 1 to 4, wherein the alkyl (meth)acrylate is methyl or ethyl (meth)acrylate.

6. The process according to any of claims 1 to 5, wherein the stabilizer is hydroquinone monomethyl ether.

7. The process according to any of claims 1 to 6, wherein the alcohol is removed from the azeotrope of entraining agent and alcohol distilled off in step (iii) by washing with water and the azeotroping agent is recycled into the reaction vessel.

8. The process according to any of claims 1 to 7, wherein, after step (v), an isosorbide ethoxylate di(meth)acrylate having a content of secondary components of < 4% by weight is obtained.

9. The process according to claim 8, wherein, after step (v), an isosorbide ethoxylate di(meth)acrylate having a content of secondary components of < 2% by weight is obtained.

10. The use of isosorbide ethoxylate di(meth)acrylate as resin component for two-pack adhesive compositions.

11. The use according to claim 10, wherein the two-pack adhesive compositions are plugging compounds for securing anchoring means in drillholes.

12. The use according to claim 10 or 11, wherein the isosorbide ethoxylate di(meth)acrylate has been prepared by the process according to any of claims 1 to 9.

## Revendications

1. Procédé pour la préparation de di(méth)acrylate d'éthoxylate d'isosorbide par transestérification d'un (méth)acrylate d'alkyle avec de l'éthoxylate d'isosorbide, comprenant les étapes :
(i) éthoxylation d'isosorbide en éthoxylate d'isosorbide,
(ii) mise en réaction d'un (méth)acrylate d'alkyle avec de l'éthoxylate d'isosorbide en présence de phosphate de calcium en tant que catalyseur et d'un stabilisant et en présence d'un agent d'entraînement qui forme un azéotrope avec l'alcool lié dans le (méth)acrylate d'alkyle,
(iii) élimination continue par distillation de l'azéotrope d'agent d'entraînement et alcool, les étapes (ii) et (iii) étant effectuées simultanément, jusqu'à ce que l'éthoxylate d'isosorbide ait pratiquement totalement réagi,
(iv) séparation du catalyseur d'avec le mélange de produits contenant du di(méth)acrylate d'éthoxylate d'isosorbide,
(v) élimination par distillation du (méth)acrylate d'alkyle n'ayant pas réagi et de l'agent d'entraînement à partir du mélange de produits.

2. Procédé selon la revendication 1, **caractérisé en ce que** l'agent d'entraînement est le (méth)acrylate d'alkyle.

3. Procédé selon la revendication 1, **caractérisé en ce que** l'agent d'entraînement est un solvant distinct, différent du (méth)acrylate d'alkyle.

4. Procédé selon la revendication 3, **caractérisé en ce que** l'agent d'entraînement est choisi dans le groupe constitué par le n-heptane et le cyclohexane.

5. Procédé selon l'une quelconque des revendications 1 à 4, **caractérisé en ce que** le (méth)acrylate d'alkyle est le (méth)acrylate de méthyle ou d'éthyle.

6. Procédé selon l'une quelconque des revendications 1 à 5, **caractérisé en ce que** le stabilisant est l'éther monométhylique d'hydroquinone.

7. Procédé selon l'une quelconque des revendications 1 à 6, **caractérisé en ce qu'**à partir de l'azéotrope d'agent d'entraînement et alcool éliminé par distillation dans l'étape (iii) on sépare l'alcool par lavage avec de l'eau et on renvoie l'agent d'entraînement dans le récipient de réaction.

8. Procédé selon l'une quelconque des revendications 1 à 7, **caractérisé en ce qu'**après l'étape (v) on obtient un di(méth)acrylate d'éthoxylate d'isosorbide ayant une teneur en composants secondaires de < 4 % en poids.

9. Procédé selon la revendication 8, **caractérisé en ce qu'**après l'étape (v) on obtient un di(méth)acrylate d'éthoxylate d'isosorbide ayant une teneur en composants secondaires de < 2 % en poids.

10. Utilisation de di(méth)acrylate d'éthoxylate d'isosorbide en tant que composant de type résine pour matières adhésives à deux composants.

11. Utilisation selon la revendication 10, **caractérisée en ce que** les matières adhésives à deux composants sont des matières de cheville pour la fixation de moyens d'ancrage dans des trous de forage.

12. Utilisation selon la revendication 10 ou 11, **caractérisée en ce que** le di(méth)acrylate d'éthoxylate d'isosorbide est préparé conformément au procédé selon l'une quelconque des revendications 1 à 9.
